# EUROPÄISCHE PATENTSCHRIFT

(11) **EP 2 552 895 B1**
(45) Veröffentlichungstag und Bekanntmachung des Hinweises auf die Patenterteilung: **25.02.2015**
(21) Anmeldenummer: 11711070.0
(22) Anmeldetag: 23.03.2011
(51) Int. Cl.: C07D 231/10

(54) **PYRAZOL-SYNTHESE DURCH KUPPLUNG VON CARBONSÄUREDERIVATEN UND ENAMINEN**
PYRAZOLE SYNTHESIS BY COUPLING OF CARBOXYLIC ACID DERIVATIVES AND ENAMINES
SYNTHÈSE DE PYRAZOLES PAR COUPLAGE D'ÉNAMINES ET DE DÉRIVÉS DE L'ACIDE CARBOXYLIQUE

(30) Priorität: 29.03.2010 DE 102010013282
(43) Veröffentlichungstag der Anmeldung: 06.02.2013
(73) Patentinhaber: Westfälische Wilhelms-Universität Münster, 48149 Münster (DE)
(72) Erfinder: NEUMANN, Julia, 40591 Düsseldorf (DE); SURI, Mamta, 48161 Münster (DE); GLORIUS, Frank, 59387 Ascheberg (DE)
(74) Vertreter: Michalski Hüttermann & Partner Patentanwälte mbB
(86) Internationale Anmeldenummer: PCT/EP2011/054484
(87) Internationale Veröffentlichungsnummer: WO 2011/120861

(56) Entgegenhaltungen:
- J. Elguero: "Pyrazoles and their Benzo Derivatives"; "4A" In: Katritzky Alan R.; Rees Charles W.: "Comprehensive Heterocyclic Chemistry", 1. Januar 1984 (1984-01-01), Pergamon Press, XP002639168, Bd. 5, Seiten 273-291, in der Anmeldung erwähnt das ganze Dokument

## Beschreibung

Die vorliegende Erfindung bezieht sich auf das Gebiet der organischen Synthese, speziell das Gebiet der Synthese von Heterocyclen.

Heterocyclische Verbindungen spielen in der organischen Chemie, speziell bei Wirkstoffen und Pharmazeutika eine immense Rolle und die Synthese von heterocyclischen Verbindungen stellt ein wichtiges und ständig wachsendes Forschungsgebiet dar.

Eine wichtige Heterocyclenklasse stellen die Pyrazole dar. Pyrazole kommen zwar in Naturstoffen relativ selten vor, sind aber ein wichtiges Strukturelement biologisch aktiver Verbindungen, welche in einigen Fällen große wirtschaftliche Bedeutung als Pharmazeutika oder Pflanzenschutzwirkstoffe besitzen. Zwei prominente Beispiele sind der COX-2-Hemmer Celecoxib, der gegen rheumatoide Arthritis eingesetzt wird, oder das Kotaktgift Fipronil^{®}, das als GABA-Inhibitor gegen Ektoparasiten wie Flöhe, Läuse, Zecken und Milben wirksam ist.

Es ist dem Fachmann bekannt (Umfassender Aufsatz über Pyrazole: L. Yet. in Comprehensive Heterocyclic Chemistry III, Vol. 4 (Eds.: A. R. Katritzky, C. A. Ramsden, E. F. V. Scriven, R. J. K. Taylor), Elsevier, 2008, 1-141), dass Pyrazole auf verschiedene Art und Weise hergestellt werden können, wobei drei Methoden am häufigsten sind:
- die Knorr-Kondensation von 1,3-Dicarbonylverbindungen mit Hydrazinen,
- die 1,3-dipolare Cycloaddition von Hydrazonen an Alkine und
- die Funktionalisierung des vorgeformten Pyrazolrings am Stickstoff durch direkte Alkylierung oder Arylierung, sei es durch nucleophile Substitution oder durch moderne Übergangsmetall-katalysierte Kohlenstoff-Stickstoff-Bindungsknüpfung.

Diese Methoden haben zwei fundamentale Nachteile: zum einen können Regioselektivitätsprobleme auftreten, sobald unterschiedliche Substituenten, speziell an den beiden α-ständigen Kohlenstoffatomen zu den Stickstoffen vorhanden sein sollen. Zum anderen benötigen sie Hydrazinsubstrate, die meist carcinogen und in einigen Fällen nicht gut verfügbar sind.

Es stellt sich somit die Aufgabe, eine verbesserte Pyrazolsynthese zu schaffen, bei der die obigen Nachteile überwunden werden können und die eine einfache, hydrazinfreie Synthese von Pyrazolen erlaubt.

Diese Aufgabe wird durch ein Verfahren nach Anspruch 1 gelöst. Demgemäß wird ein Verfahren zur Herstellung von Pyrazolen durch Umsetzung von Enaminen und N-haltigen Carbonsäurederivaten in Gegenwart eines Oxidationsmittels vorgeschlagen.

Überraschenderweise hat sich herausgestellt, dass Enamine und N-haltige Carbonsäurederivate in Gegenwart eines Oxidationsmittels zu Pyrazolen reagieren. Dieses Verfahren bietet bei vielen Anwendungen insbesondere einen oder mehrere der folgenden Vorteile:
- Die Reagentien sind stabile, gängige Verbindungen, die einfach zugänglich sind
- Das Verfahren toleriert viele sonstige funktionelle Gruppen
- Dadurch, dass die "N-N"-Bindung geschlossen wird, kann eine Kontrolle der Regioisomere (insbesondere am C-3 und C-5) auf einfache Weise erfolgen.
- Aufgrund der einfachen Reaktionsführung eignet sich das Verfahren auch für eine technische Anwendung, es kann zur Synthese von Pyrazolen sowohl in Milligrammals auch in Multigramm und Kilogramm-Mengen dienen.
- Die gemäß der vorliegenden Erfindung zugänglichen Pyrazolderivate können für die Synthese zahlreicher Folgeprodukte genutzt werden, die z. B. Bedeutung als Pharmaka für die Human- und Veterinärmedizin oder als Pflanzenschutzwirkstoffe besitzen.
- Das Verfahren ist robust und nicht luftempfindlich, benötigt daher keine Schutzgasatmosphäre.
- Durch die Modularität der Reaktion kann durch Variation einer oder mehrerer Komponenten leicht das Substitutionsmuster variiert werden und dadurch Verbindungsbibliotheken mit systematisch variierten Resten erzeugt werden.
- In einem Ein-Topf-Verfahren können aus einfachen, kommerziell erhältlichen Bausteinen (Amin, Keton und Nitril) Pyrazole unmittelbar aufgebaut werden.

Der Term "Umsetzung" im Sinne der vorliegenden Erfindung bedeutet insbesondere, dass die jeweiligen Substrate mit sich und mit einem Oxidationsmittel in Kontakt gebracht werden. In der Regel geschieht dies durch Vermischen oder Suspendieren. Es sei angemerkt, dass je nach konkreter Ausführung des Verfahrens entweder die beiden Edukte vorgelegt und anschließend oxidiert wird; es kann aber auch günstig sein, ein Edukt zusammen mit dem Oxidationsmittel vorzulegen und das andere zuzugeben.

Gemäß einer bevorzugten Ausführungsform der Erfindung liegt die Reaktionstemperatur zwischen ≥0 und ≤200°C, bevorzugt zwischen ≥60 und ≤140 °C und besonders bevorzugt zwischen ≥80 und ≤120 °C.

Sowohl Druck als auch Art der Gasatmosphäre über der Reaktion sind nicht kritisch und verschiedene Gase und Drücke sind geeignet, z. B. Luft, Stickstoff, Argon, Sauerstoff, bevorzugt Luft und Stickstoff. Die Reaktion kann bei verschiedenen Drücken durchgeführt werden, bevorzugt bei Drücken von 1 bis 10 atm, besonders bevorzugt bei 1 bis 4 atm. Die Erhöhung des Drucks kann zu einer Erhöhung des Siedepunkts führen, was eine Beschleunigung der Reaktion bewirken kann.

Gegenstand der Erfindung sind weiterhin Verfahren, in denen das Reaktionsprodukt bereits unter den Reaktionsbedingungen oder durch schrittweise Variation der Reaktionsbedingungen weiterreagiert, z. B. durch eine Verseifung einer Estergruppe, eine Decarboxylierungsreaktion oder eine Hydrierung.

Die Aufarbeitung der Reaktionsgemische und die Reinigung der Produkte sind üblicherweise weniger kritisch und richten sich nach den jeweiligen physikalischen Eigenschaften der erzeugten Produkte und Nebenprodukte. Bevorzugte Aufarbeitungs- und Reinigungsmethoden sind Destillation, Sublimation, Kristallisation, Chromatographie, Filtration und Extraktion. Dadurch, dass Hydrazine vermieden werden, ist eine Aufreinigung im Vergleich zu den bisherigen Reaktionsverfahren meist einfacher und unter Erhalt eines Produktes in größerer Reinheit möglich.

Gemäß einer bevorzugten Ausführungsform der vorliegenden Erfindung beträgt das Verhältnis von Enaminen zu N-haltigen-Carbonsäurederivaten von (mol N-haltige Carbonsäurederivate:mol Enamin) ≥0.2:1 bis ≤150:1, bevorzugt ≥1:1 bis ≤100:1, sowie am meisten bevorzugt von ≥2:1 bis ≤20:1. Der Überschuss an N-haltigem Carbonsäurederivat kann u.a. dadurch erfolgen, dass die Reaktion im N-haltigem Carbonsäurederivat als Lösemittel erfolgt. In diesem Fall sind bevorzugte Verbindungen insbesondere Acetonitril, Propionitril, Isobutyronitril, Butyronitril, Valeronitril, Capronitril, Heptansäurenitril, Octansäurenitril, Nonansäurenitril, Benzonitril, sowie ihrer mono- oder mehrfach fluorierten, geradkettigen und verzweigten Derivate.

Gemäß einer bevorzugten Ausführungsform der vorliegenden Erfindung findet die Reaktion in einem Lösemittel statt, ausgewählt aus der Gruppe enthaltend Pentan, Hexan, Heptan, Octan, Petrolether, Toluol, Xylole, Chlorbenzol, o-Dichlorbenzol, Ethylacetat, Tetrahydrofuran, Diethylether, Methyltertbutylether, 1,4-Dioxan, N,N-Dimethylformamid, Dimethylsulfoxid, 1,2-Dichlorethan, N,N-Dimethylacetamid, Dimethoxyethan oder Carbonsäuren wie Ameisensäure, Essigsäure, Trifluoressigsäure, Chloressigsäure, Trichloressigsäure, Propionsäure, Buttersäure, oder Alkohole wie Methanol, Ethanol, Propanol, Isopropanol, Butanol, t-Butanol oder Phenol, bevorzugt Ethylacetat, Tetrahydrofuran, Diethylether, Methyltertbutylether, 1,4-Dioxan, N,N-Dimethylformamid, Dimethylsulfoxid, 1,2-Dichlorethan, N,N-Dimethylacetamid, t-Butanol, Chlorbenzol, Toluol, Dimethoxyethan, Hexan, o-Dichlorbenzol, besonders bevorzugt 1,4-Dioxan, N,N-Dimethylformamid, Dimethylsulfoxid, 1,2-Dichlorethan, N,N-Dimethylacetamid, t-Butanol und Chlorbenzol oder Mischungen daraus.

Gemäß einer bevorzugten Ausführungsform der vorliegenden Erfindung sind die N-haltigen Carbonsäurederivate ausgewählt aus der Gruppe enthaltend Nitrile, Carbonsäureamide, Amidine, Imidate, Imidoylchloride, abgeleitete protonierte Salze dieser Verbindungen sowie Mischungen daraus.

Bevorzugt werden als Enamine Verbindungen der folgenden Struktur eingesetzt: 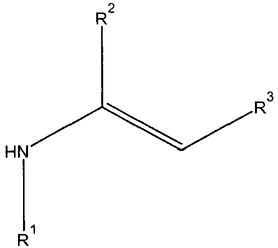 wobei R¹, R² und R³ unabhängig voneinander ausgewählt sind aus der Gruppe enthaltend Wasserstoff, Hydroxyl, Halogen, Pseudohalogen, Formyl, Carboxy- und/oder Carbonyl derivaten, Alkyl, langkettiges Alkyl, Alkoxy, langkettiges Alkoxy, Cycloalkyl, Halogenalkyl, Aryl, Arylene, Halogenaryl, Heteroaryl, Heteroarylene, Heterocycloalkylene, Heterocycloalkyl, Halogenheteroaryl, Alkenyl, Halogenalkenyl, Alkinyl, Halogenalkinyl, Keto, Ketoaryl, Halogenketoaryl, Ketoheteroaryl, Ketoalkyl, Halogenketoalkyl, Ketoalkenyl, Halogenketoalkenyl, Phosphoalkyl, Phosphonate, Phosphate, Phosphin, Phosphine oxid, Phosphoryl, Phosphoaryl, Sulphonyl, Sulphoalkyl, Sulphoarenyl, Sulphonate, Sulphate, Sulphone, Amine, Polyether, Silylalkyl, Silylalkyloxy, wobei bei geeigneten Resten eine oder mehrere nicht-benachbarte CH₂-Gruppen unabhängig voneinander durch -O-, -S-, -NH-,-NR°-, -SiR°R°°-, -CO-, -COO-, -OCO-, -OCO-O-, -SO₂-, -S-CO-, -CO-S-, -CY1=CY2 oder-C=C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)

Bevorzugt werden als N-haltigen Carbonsäurederivate Verbindung mit folgenden Strukturen eingesetzt: 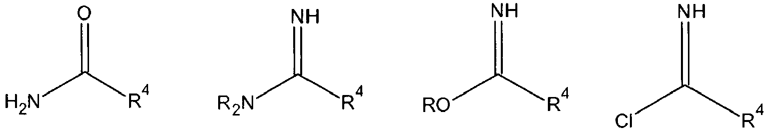 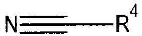 wobei R ausgewählt ist aus Alkyl, Aryl, Halogenalkyl und R⁴ ausgewählt ist aus der Gruppe enthaltend Wasserstoff, Hydroxyl, Halogen, Pseudohalogen, Formyl, Carboxy- und/oder Carbonyl derivaten, Alkyl, langkettiges Alkyl, Alkoxy, langkettiges Alkoxy, Cycloalkyl, Halogenalkyl, Aryl, Arylene, Halogenaryl, Heteroaryl, Heteroarylene, Heterocycloalkylene, Heterocycloalkyl, Halogenheteroaryl, Alkenyl, Halogenalkenyl, Alkinyl, Halogenalkinyl, Keto, Ketoaryl, Halogenketoaryl, Ketoheteroaryl, Ketoalkyl, Halogenketoalkyl, Ketoalkenyl, Halogenketoalkenyl, Phosphoalkyl, Phosphonate, Phosphate, Phosphin, Phosphine oxid, Phosphoryl, Phosphoaryl, Sulphonyl, Sulphoalkyl, Sulphoarenyl, Sulphonate, Sulphate, Sulphone, Amine, Polyether, Silylalkyl, Silylalkyloxy, wobei bei geeigneten Resten eine oder mehrere nicht-benachbarte CH₂-Gruppen unabhängig voneinander durch -O-, -S-, -NH-,-NR°-, -SiR°R°°-, -CO-, -COO-, -OCO-, -OCO-O-, -SO₂-, -S-CO-, -CO-S-, -CY1=CY2 oder-C≡C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)

allgemeine Gruppendefinition: Innerhalb der Beschreibung und den Ansprüchen werden allgemeine Gruppen, wie z.B: Alkyl, Alkoxy, Aryl etc. beansprucht und beschrieben. Wenn nicht anders beschrieben, werden bevorzugt die folgenden Gruppen innerhalb der allgemein beschriebenen Gruppen im Rahmen der vorliegenden Erfindung verwendet:
alkyl: lineare und verzweigte C1-C8-Alkyle,
langkettige Alkyle: lineare und verzweigte C5-C20 Alkyle
alkenyl: C2-C6-alkenyl,
cycloalkyl: C3-C8-cycloalkyl,
alkoxy: C1-C6-alkoxy,
langkettig Alkoxy: lineare und verzweigte C5-C20 Alkoxy
alkylene: ausgewählt aus der Gruppe enthaltend: methylene; 1,1-ethylen; 1,2-ethylene; 1,1-propylidene; 1,2-propylene; 1,3- propylene; 2,2-propylidene; butan-2-ol-1,4-diyl; propan-2-ol-1,3-diyl; 1, 4-butylene; cyclohexane-1,1-diyl; cyclohexan-1,2-diyl; cyclohexan-1,3- diyl; cyclohexan-1,4-diyl; cyclopentane-1,1-diyl; cyclopentan-1,2-diyl; und cyclopentan-1,3-diyl,
aryl: ausgewählt aus Aromaten mit einem Molekulargewicht unter 300Da.
arylene: ausgewählt aus der Gruppe enthaltend: 1,2-phenylene; 1,3- phenylene; 1,4-phenylene; 1,2-naphtalenylene; 1,3-naphtalenylene; 1,4- naphtalenylene; 2,3-naphtalenylene; 1-hydroxy-2,3-phenylene; 1-hydroxy-2,4-phenylene; 1-hydroxy-2,5- phenylene; und 1-hydroxy-2,6-phenylene,
heteroaryl: ausgewählt aus der Gruppe enthaltend: pyridinyl; pyrimidinyl; pyrazinyl; triazolyl; pyridazinyl; 1,3,5-triazinyl; chinoninyl; isochinoninyl; chinoxalinyl; imidazolyl; pyrazolyl;
benzimidazolyl; thiazolyl; oxazolidinyl; pyrrolyl; thiophenyl; carbazolyl; indolyl; und isoindolyl, wobei das Heteroaryl mit der Verbindung über jedes Atom im Ring des ausgewählten Heteroaryls verbunden sein kann.
heteroarylene: ausgewählt aus der Gruppe enthaltend: pyridindiyl; quinolindiyl; pyrazodiyl; pyrazoldiyl; triazolediyl; pyrazindiyl, thiophendiyl; und imidazolediyl, wobei das heteroarylene als Brücke in der Verbindung über ein beliebiges Atom im Ring des ausgewählten Heteroaryls fungiert, speziell bevorzugt sind: pyridin-2, 3-diyl; pyridin-2,4-diyl; pyridin-2,5-diyl; pyridin-2,6-diyl; pyridin-3,4- diyl; pyridin-3,5-diyl; quinolin-2,3-diyl; quinolin-2,4-diyl; quinolin-2, 8-diyl; isoquinolin-1,3-diyl; isoquinolin-1,4-diyl; pyrazol-1,3-diyl; pyrazol-3,5- diyl; triazole-3,5-diyl; triazole-1,3-diyl; pyrazin-2,5-diyl; und imidazole-2,4-diyl, thiophen-2,5-diyl, thiophen-3,5-diyl; ein -C1-C6-heterocycloalkyl, ausgewählt aus der Gruppe enthaltend: piperidinyl; piperidine; 1,4-piperazine, tetrahydrothiophene; tetrahydrofuran; 1,4,7-triazacyclononane; 1,4,8,11-tetraazacyclotetradecane; 1,4,7,10,13-pentaazacyclopentadecane; 1,4-diaza- 7-thia-cyclononane; 1,4- diaza-7-oxa-cyclononane; 1,4,7,10-tetraazacyclododecane; 1,4-dioxane; 1,4, 7-trithia-cyclononane; pyrrolidine; und tetrahydropyran, wobei das Heteroaryl mit dem C1-C6-Alkyl über jedes Atom im Ring des ausgewählten Heteroaryls verbunden sein kann.
heterocycloalkylene: ausgewählt aus der Gruppe enthaltend: piperidin-1,2- ylene; piperidin-2,6-ylene; piperidin-4,4-ylidene; 1,4-piperazin-1,4-ylene; 1,4-piperazin-2,3-ylene; 1,4-piperazin-2,5-ylene; 1,4-piperazin-2,6-ylene; 1,4-piperazin- 1,2-ylene; 1,4-piperazin-1,3-ylene; 1,4-piperazin-1,4-ylene; tetrahydrothiophen-2,5-ylene; tetrahydrothiophen-3,4-ylene; tetrahydrothiophen-2,3-ylene; tetrahydrofuran-2,5-ylene; tetrahydrofuran- 3,4-ylene; tetrahydrofuran-2,3-ylene; pyrrolidin-2,5-ylene; pyrrolidin-3,4-ylene; pyrrolidin-2,3-ylene; pyrrolidin-1,2-ylene; pyrrolidin-1,3-ylene; pyrrolidin-2,2-ylidene; 1,4,7-triazacyclonon-1,4-ylene; 1,4,7- triazacyclonon-2,3-ylene; 1,4,7-triazacyclonon-2,9-ylene; 1,4,7-triazacyclonon-3,8-ylene; 1,4,7-triazacyclonon-2,2- ylidene; 1,4,8,11-tetraazacyclotetradec-1,4-ylene;
1,4,8,11- tetraazacyclotetradec-1,8-ylene-, 1,4,8,11-tetraazacyclotetradec-2,3-ylene; 1,4,8,11-tetraazacyclotetradec-2,5-ylene; 1,4,8,11-tetraazacyclotetradec-1,2-ylene; 1,4,8,11-tetraazacyclotetradec-2,2-ylidene; 1,4,7,10-tetraazacyclododec-1,4-ylene; 1,4,7,10-tetraazacyclododec-1,7-ylene; 1,4,7,10-tetraazacyclododec-1,2- ylene; 1,4,7,10-tetraazacyclododec-2,3- ylene; 1,4,7,10-tetraazacyclododec-2,2-ylidene; 1,4,7,10,13 pentaazacyclopentadec-1,4-ylene; 1,4,7,10,13- pentaazacyclopentadec-1,7-ylene; 1,4,7,10,13-pentaazacyclopentadec-2,3- ylene; 1,4,7,10,13-pentaazacyclopentadec-1,2-ylene; 1,4,7,10, 13-pentaazacyclopentadec-2,2-ylidene; 1,4-diaza-7-thia-cyclonon- 1,4-ylene; 1,4-diaza-7-thia-cyclonon-1,2-ylene; 1,4-diaza-7thia-cyclonon- 2,3-ylene; 1,4-diaza-7-thia-cyclonon-6,8-ylene; 1,4-diaza-7-thia-cyclonon- 2,2-ylidene; 1,4-diaza-7-oxacyclonon-1,4-ylene; 1,4-diaza-7-oxa-cyclonon- 1,2-ylene; 1,4diaza-7-oxa-cyclonon-2,3-ylene; 1,4-diaza-7-oxa-cyclonon-6, 8-ylene; 1,4-diaza-7-oxa-cyclonon-2,2-ylidene; 1,4-dioxan-2,3-ylene; 1,4-dioxan-2,6-ylene; 1,4-dioxan-2,2-ylidene; tetrahydropyran-2,3-ylene; tetrahydropyran-2,6-ylene; tetrahydropyran-2,5-ylene; tetrahydropyran-2,2- ylidene; 1,4,7-trithia-cyclonon-2,3-ylene; 1,4,7-trithia-cyclonon-2,9- ylene; und 1,4,7-trithia-cyclonon-2,2-ylidene,
helerocycloalkyl: ausgewählt aus der Gruppe enthaltend: pyrrolinyl; pyrrolidinyl; morpholinyl; piperidinyl; piperazinyl; hexamethylene imine; 1,4-piperazinyl; tetrahydrothiophenyl; tetrahydrofuranyl; 1,4,7- triazacyclononanyl; 1,4,8,11-tetraazacyclotetradecanyl; 1,4,7,10,13- pentaazacyclopentadecanyl; 1,4-diaza-7-thiacyclononanyl; 1,4-diaza-7-oxa- cyclononanyl; 1,4,7,10-tetraazacyclododecanyl; 1,4-dioxanyl; 1,4,7- trithiacyclononanyl; tetrahydropyranyl; und oxazolidinyl, wobei das Heterocycloalkyl mit der Verbindung über jedes Atom im Ring des ausgewählten Heterocycloalkyls verbunden sein kann.
amine: die Gruppe -N(R)2 wobei jedes R unabhängig ausgewählt ist aus: Wasserstoff; C1-C6-alkyl; C1-C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet
halogen: ausgewählt aus der Gruppe enthaltend: F; Cl; Br und I,
halogenalkyl: ausgewählt aus der Gruppe enthaltend mono, di, tri-, poly und perhalogenated lineare und verzweigte C1-C8-allcyl
pseudohalogen: ausgewählt aus der Gruppe enthaltend -CN, -SCN, -OCN, N3, -CNO, -SeCN
sulphonate: die Gruppe -S(O)20R, wobei R ausgewählt ist aus: Wasserstoff; C1- C6-alkyl; phenyl; C1-C6-alkyl-C6H5; Li; Na; K; Cs; Mg; und Ca,
sulphate: die Gruppe -OS(O)2OR, wobei R ausgewählt ist aus: Wasserstoff; C1- C6-alkyl; phenyl; C1-C6-alkyl-C6H5; Li; Na; K; Cs; Mg; und Ca,
sulphone: die Gruppe -S(O)2R, wobei R ausgewählt ist aus: Wasserstoff; C1-C6- alkyl; phenyl; C1-C6-alkyl-C6H5 und amine (to give sulphonamide) ausgewählt aus der Gruppe: - NR'2, wobei jedes R' unabhängig ausgewählt ist aus: Wasserstoff; C1-C6-alkyl; C1C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet
carboxylat: die Gruppe -C(O)OR, wobei R ausgewählt ist aus: Wasserstoff; C1-C6-alkyl; phenyl; C1-C6-alkyl-C6H5; Li; Na; K; Cs; Mg; und Ca,
carbonyl: die Gruppe -C(O)R, wobei R ausgewählt ist aus: Wasserstoff; C1-C6-alkyl; phenyl; C1-C6-alkyl-C6H5 und amine (to give amide) ausgewählt aus der Gruppe: -NR'2, wobei jedes R' unabhängig ausgewählt ist aus: Wasserstoff; C1- C6-alkyl; C1-C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet
phosphonate: die Gruppe -P(O) (OR) 2, wobei jedes R unabhängig ausgewählt ist aus: Wasserstoff; C1-C6-alkyl; phenyl; C1-C6-alkyl-C6H5; Li; Na; K; Cs; Mg; und Ca,
phosphate: die Gruppe -OP(O)(OR)2, wobei jedes R unabhängig ausgewählt ist aus: Wasserstoff; C1-C6-alkyl; phenyl; C1-C6-alkyl-C6H5; Li; Na; K; Cs; Mg; und Ca,
phosphine: die Gruppe -P(R)2, wobei jedes R unabhängig ausgewählt ist aus: Wasserstoff; C1-C6-alkyl; phenyl; und C1-C6-alkyl-C6H5,
phosphine oxid: die Gruppe -P (O) R2, wobei R unabhängig ausgewählt ist aus: Wasserstoff; C1-C6-alkyl; phenyl; und C1-C6-alkyl-C6H5; und amine (to give phosphonamidate) ausgewählt aus der Gruppe: -NR'2, wobei jedes R' unabhängig ausgewählt ist aus: Wasserstoff; C1-C6-alkyl; C1-C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet.
polyether: ausgewählt aus der Gruppe enthaltend -(O-CH₂-CH(R))ₙ-OH und -(O-CH₂-CH(R))ₙ-H wobei R unabhängig ausgewählt ist aus: Wasserstoff, alkyl, aryl, halogen und n ist von 1 to 250
silylalkyl: die Gruppe -SiR₃, wobei jedes R unabhängig voneinander ausgewählt ist aus: Wasserstoff; C1-C6- alkyl; phenyl; C1-C6-alkyl-C6H5 und amine (to give sulphonamide) ausgewählt aus der Gruppe: -NR'2, wobei jedes R' unabhängig ausgewählt ist aus:
   Wasserstoff; C1-C6-alkyl; C1C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet
silylalkyloxy: die Gruppe -OSiR₃, wobei jedes R unabhängig voneinander ausgewählt ist aus: Wasserstoff; C1-C6- alkyl; phenyl; C1-C6-alkyl-C6H5 und amine (to give sulphonamide) ausgewählt aus der Gruppe: -NR'2, wobei jedes R' unabhängig ausgewählt ist aus: Wasserstoff; C1-C6-alkyl; C1C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet
Soweit nicht anders erwähnt, sind die folgenden Gruppen mehr bevorzugte Gruppen innerhalb der allgemeinen Gruppendefinition:
   alkyl: lineare und verzweigte C1-C6-alkyl,
   langkettige Alkyle: lineare und verzweigte C5-C10 alkyl, vorzugsweise C6-C8 alkyle
   alkenyl: C3-C6-alkenyl,
   cycloalkyl: C6-C8-cycloalkyl,
   alkoxy: Cl-C4-alkoxy,
   langkettig Alkoxy: lineare und verzweigte C5-C10 alkoxy, vorzugsweise lineare C6-C8 alkoxy
   alkylene: ausgewählt aus der Gruppe enthaltend: methylene; 1,2-ethylene; 1,3-propylene; butan-2-ol-1,4-diyl; 1,4-butylene; cyclohexane-1,1-diyl; cyclohexan-1,2-diyl; cyclohexan-1,4-diyl; cyclopentane-1,1-diyl; und cyclopentan-1,2-diyl,
   aryl: ausgewählt aus der Gruppe enthaltend: phenyl; biphenyl; naphthalenyl; anthracenyl; und phenanthrenyl,
   arylene: ausgewählt aus der Gruppe enthaltend: 1,2-phenylene; 1,3- phenylene; 1,4-phenylene; 1,2-naphtalenylene; 1,4-naphtalenylene; 2,3- naphtalenylene und 1-hydroxy-2,6-phenylene,
heteroaryl: ausgewählt aus der Gruppe enthaltend:
   pyridinyl; pyrimidinyl; chinoninyl; pyrazolyl; triazolyl; isochinoninyl; imidazolyl; und oxazolidinyl, wobei das Heteroaryl mit der Verbindung über jedes Atom im Ring des ausgewählten Heteroaryls verbunden sein kann,
   heteroarylene: ausgewählt aus der Gruppe enthaltend: pyridin 2,3-diyl; pyridin-2,4-diyl; pyridin-2,6-diyl; pyridin-3,5-diyl; quinolin-2,3-diyl; quinolin-2,4-diyl; isoquinolin-1,3-diyl; isoquinolin-1,4-diyl; pyrazol-3,5-diyl; und imidazole-2,4-diyl,
heterocycloalkyl: ausgewählt aus der Gruppe enthaltend:
   pyrrolidinyl; morpholinyl; piperidinyl; piperidinyl; 1,4 piperazinyl; tetrahydrofuranyl; 1,4,7-triazacyclononanyl; 1,4,8,11-tetraazacyclotetradecanyl; 1,4,7,10,13-pentaazacyclopentadecanyl; 1,4,7,10-tetraazacyclododecanyl; und piperazinyl, wobei das Heteroaryl mit der Verbindung über jedes Atom im Ring des ausgewählten Heteroaryls verbunden sein kann
heterocycloalkylene: ausgewählt aus der Gruppe enthaltend:
   piperidin-2,6-ylene; piperidin-4,4-ylidene; 1,4-piperazin-1,4-ylene; 1,4-piperazin-2,3-ylene; 1,4-piperazin-2,6-ylene; tetrahydrothiophen-2,5-ylene; tetrahydrothiophen-3,4-ylene; tetrahydrofuran-2,5-ylene; tetrahydrofuran-3,4-ylene; pyrrolidin-2,5-ylene; pyrrolidin-2,2-ylidene; 1,4,7-triazacyclonon-1,4-ylene; 1,4,7-triazacyclonon-2,3-ylene; 1,4,7-triazacyclonon-2,2-ylidene; 1,4,8,11- tetraazacyclotetradec-1,4-ylene; 1,4,8,11-tetraazacyclotetradec-1,8-ylene; 1,4,8,11-tetraazacyclotetradec-2,3-ylene; 1,4,8,11-tetraazacyclotetradec-2,2-ylidene; 1,4,7,10-tetraazacyclododec-1,4-ylene; 1,4,7,10-tetraazacyclododec-1,7-ylene; 1,4,7,10-tetraazacyclododec-2,3-ylene; 1,4,7,10-tetraazacyclododec-2,2-ylidene; 1,4,7,10,13- pentaazacyclopentadec-1,4-ylene; 1,4,7,10,13-pentaazacyclopentadec-1,7-ylene; 1,4-diaza-7-thia-cyclonon-1,4 ylene; 1,4-diaza-7-thia-cyclonon-2,3-ylene; 1,4-diaza-7-thiein cyclonon-2,2-ylidene; 1,4-diaza-7-oxa-cyclonon-1,4-ylene; 1,4 diaza-7-oxa-cyclonon-2,3-ylene;1,4-diaza-7-oxa-cyclonon-2,2- ylidene; 1,4-dioxan-2,6-ylene; 1,4-dioxan-2,2-ylidene; tetrahydropyran-2,6-ylene; tetrahydropyran-2,5-ylene; und tetrahydropyran- 2,2-ylidene, ein -C1-C6-alkyl-heterocycloalkyl, wobei das Heterocycloalkyl ausgewählt aus der Gruppe enthaltend: piperidinyl; 1,4-piperazinyl; tetrahydrofuranyl; 1,4,7- triazacyclononanyl; 1,4,8,11-tetraazacyclotetradecanyl; 1,4,7,10,13-pentaazacyclopentadecanyl; 1,4,7,10-tetraazacyclododecanyl; und pyrrolidinyl, wobei das Heterocycloalkyll mit der Verbindung über jedes Atom im Ring des ausgewählten Heterocycloalkyls verbunden sein kann
amine: die Gruppe -N (R) 2, wobei jedes R unabhängig ausgewählt ist aus: Wasserstoff; C1-C6-alkyl; und benzyl,
halogen: ausgewählt aus der Gruppe enthaltend: F und Cl,
sulphonate: die Gruppe -S(O)20R, wobei R ausgewählt ist aus: Wasserstoff; C1- C6-alkyl; Na; K; Mg; und Ca,
sulphate: die Gruppe -OS(O)2OR, wobei R ausgewählt ist aus: Wasserstoff; C1- C6-alkyl; Na; K; Mg; und Ca,
sulphone: die Gruppe -S(O)2R, wobei R ausgewählt ist aus: Wasserstoff; C1-C6- alkyl; benzyl und amine ausgewählt aus der Gruppe: -NR'2, wobei jedes R' unabhängig ausgewählt ist aus:
   Wasserstoff; C1-C6-alkyl; und benzyl,
carboxylat: die Gruppe -C(O)OR, wobei R ausgewählt ist aus Wasserstoff; Na; K; Mg; Ca; C1-C6-alkyl; und benzyl,
carbonyl: die Gruppe: -C(O)R, wobei R ausgewählt ist aus: Wasserstoff; C1-C6-alkyl; benzyl und amine ausgewählt aus der Gruppe: -NR'2, wobei jedes R' unabhängig ausgewählt ist aus: Wasserstoff; C1-C6-alkyl; und benzyl,
phosphonate: die Gruppe -P(O) (OR)2, wobei jedes R unabhängig ausgewählt ist aus: Wasserstoff; C1-C6-alkyl; benzyl; Na; K; Mg; und Ca,
phosphate: die Gruppe -OP(O) (OR)2, wobei jedes R unabhängig ausgewählt ist aus: Wasserstoff; C1-C6-alkyl; benzyl; Na; K; Mg; und Ca,
phosphine: die Gruppe -P(R)2, wobei jedes R unabhängig ausgewählt ist aus: Wasserstoff; C1-C6-alkyl; und benzyl,
phosphine oxid: die Gruppe -P(O)R2, wobei R unabhängig ausgewählt ist aus: Wasserstoff; C1-C6-alkyl; benzyl und amine ausgewählt aus der Gruppe: -NR'2, wobei jedes R' unabhängig ausgewählt ist aus: Wasserstoff; C1-C6-alkyl; und benzyl.
polyether: ausgewählt aus der Gruppe enthaltend-(O-CH₂-CH(R))ₙ-OH und -(O-CH₂-CH(R))ₙ-H wobei R unabhängig ausgewählt ist aus: Wasserstoff, methyl, halogen und n ist von 5 bis 50, bevorzugt 10 bis 25.
M, Mₙ (n ist eine ganze Zahl) : Metalle, wobei zwei Metalle M unabhängig voneinander ausgewählt sind, wenn nicht anders angezeigt.

Bevorzugt werden Enamine der folgenden Struktur verwendet: 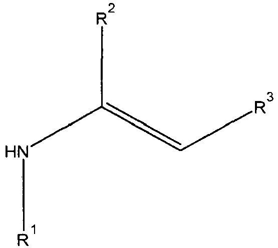 wobei R¹ und R² wie oben beschrieben sind, R³ aber eine elektronenziehende Gruppe enthält, d.h. ausgewählt ist aus der Gruppe enthaltend Formyl, Carboxy- und/oder Carbonylderivate, Keto, Ketoaryl, Halogenketoaryl, Ketoheteroaryl, Ketoalkyl, Halogenketoalkyl, Ketoalkenyl, Halogenketoalkenyl, Phosphoalkyl, Phosphonate, Phosphate, Phosphin, Phosphinoxid, Phosphoryl, Phosphoaryl, Sulphonyl, Sulphoalkyl, Sulphoarenyl, Sulphonate, Sulphate, Sulphone, Amine, Polyether, Silylalkyl, Silylalkyloxy, wobei bei geeigneten Resten eine oder mehrere nicht-benachbarte CH₂-Gruppen unabhängig voneinander durch -O-, -S-, -NH-,-NR°-, -SiR°R°°-, -CO-, -COO-, -OCO-, -OCO-O-, -SO₂-, -S-CO-, -CO-S-, -CY1=CY2 oder-C≡C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)

Verbindungen dieses Typs haben sich in der Praxis als besonders günstig herausgestellt, da zum einen die so erhaltenen Enamine meist deutlich stabiler sind und zum anderen oftmals die Reaktivität größer ist.

Es sei an dieser Stelle angemerkt, dass die erfindungsgemäßen Enamine je nach Ausführungsform entweder in Substanz eingesetzt werden oder während der *Umsetzung in situ* hergestellt werden. Dies kann (je nach Ausführungsform) durch Umsetzung von geeigneten Aminen mit Ketonen (ggf. unter Verwendung wasserentziehender Substanzen wie etwa TiCl₄) oder (wenn ein Enamin der obigen Struktur mit einer elektronenziehenden Gruppe verwendet wird) durch Michael-Addition eines Amins an ein Alkin erfolgen.

Ebenso sei an dieser Stelle angemerkt, dass - falls Nitrile als N-haltigen Carbonsäurederivate gewählt werden - diese entweder in Substanz eingesetzt werden können oder aus anderen Carbonsäurederivaten oder geeigneten Vorläufersubstanzen *in situ* während der Reaktion hergestellt werden können. Geeignete Vorläufersubstanzen sind insbesondere diese, die bei wasserentziehenden Bedingungen Nitrile ergeben. In diesem Fall kann - falls gewünscht - sowohl das Enamin wie das Nitril *in situ* während des Verfahrens entstehen.

Gemäß einer bevorzugten Ausführungsform der Erfindung wird die Umsetzung in Gegenwart einer Lewis-Säure durchgeführt. Dies hat sich oftmals als sehr reaktionsbeschleunigend herausgestellt. Bevorzugt ist die Lewis-Säure ausgewählt aus der Gruppe enthaltend Metallionen, bevorzugt mit Cu(II)-, Zn(II)-, Al(III)-, In(III)-Salzen, sowie Protonensäuren. Besonders bevorzugt sind Metallsalze, dabei insbesondere Cu(II)-Salze, bevorzugt Cu(OAc)₂ oder Cu(OAc)₂-Hydrat.

Gemäß einer bevorzugten Ausführungsform der Erfindung wird die Umsetzung in Gegenwart von Kupferionen durchgeführt.

Die Lewis-Säure, die als Katalysator wirkt, ist bevorzugt in einem Anteil von ≥0,1% (mol Katalysator:mol Enamin) bezogen auf das Enamin eingesetzt. Für den Fall, dass neben der Lewis-Säure noch ein Oxidationsmittel vorhanden ist (vgl. unten) sind weiter bevorzugte Bereiche ≥1% bis ≤20%, noch bevorzugt ≥2% bis ≤10%.

Gemäß einer bevorzugten Ausführungsform ist das Oxidationsmittel ausgewählt aus der Gruppe enthaltend Sauerstoff (bzw. Luft), Ozon, Hypohalogenide, insbesondere Hypochlorit, Peroxide, Metalle bzw. Metallsalze oder Mischungen daraus.

Besonders bevorzugt sind Metallsalze, dabei insbesondere Cu(II)-Salze, bevorzugt Cu(OAc)₂ oder Cu(OAc)₂-Hydrat.

Das Oxidationsmittel wird bevorzugt in einem Verhältnis (bezogen auf das Enamin) von ≥1.5:1 bis ≤30:1 (mol Oxidationsmittel : mol Enamin) eingesetzt, noch bevorzugt ist ein Verhältnis von ≥2:1 bis ≤10:1, noch bevorzugt ≥2.5:1 bis ≤3:1

Besonders bevorzugt ist der Einsatz von Cu(II)-Salzen, insbesondere aus dem Grund, dass das Kupfer gleichzeitig als Lewis-Säure agieren kann und somit oxidierend wie katalytisch aktiv wirkt.

Die vorliegende Erfindung ist aber nicht darauf beschränkt, sondern es kann genauso ein Oxidationsmittel neben einer nicht-oxidativ wirkenden Lewis-Säure eingesetzt werden.

Für den Fall, dass Sauerstoff (bzw. Luft) als Oxidationsmittel eingesetzt wird und die Reaktion in Gegenwart einer unterstöchiometrischen Lewis-Säure, welche Metallionen enthält - bevorzugt Cu(II) - durchgeführt wird, hat sich in der Praxis herausgestellt, dass die Ausbeuten durch Zusatz einer in α-Stellung tertiären oder sekundären Carbonsäure nochmals erhöht werden können. Dies ist somit eine bevorzugte Ausführungsform der vorliegenden Erfindung. Bevorzugt sind dabei α-tertiäre Carbonsäuren wie z.B. Pivalinsäure. Das Konzentration beträgt dabei (in mol-% bezogen auf mol Lewis Säure) bevorzugt >0% bis 100%, bevorzugt ≥30% bis ≤70%, noch bevorzugt ≥40% bis ≤60% sowie am meisten bevorzugt etwa 50%.

Die vorgenannten sowie die beanspruchten und in den Ausführungsbeispielen beschriebenen erfindungsgemäß zu verwendenden Bauteile unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können.

Weitere Einzelheiten, Merkmale und Vorteile des Gegenstandes der Erfindung ergeben sich aus den Unteransprüchen sowie aus der nachfolgenden Beschreibung der zugehörigen Beispiele, in denen - beispielhaft - mehrere Ausführungsbeispiele des erfindungsgemäßen Verfahrens dargestellt sind. Diese sind rein illustrativ zu verstehen.

### Allgemeine Versuchsvorschrift A (AVV A)

In ein über Nacht im Ofen (140 °C) gelagertes Schraubdeckelgläschen (mit PTFE-Dichtungsscheibe, Volumen: ca. 7 mL) mit Magnetrührstäbchen wurde an der Luft das Enamin-Startmaterial (1.0 mmol; (Z)-3-(4-Fluor-phenylamino)-but-2-ensäuremethylester im Fall von Beispiel 1) sowie Cu(OAc)₂ (3.0 mmol) eingewogen. Bei flüssigen oder öligen Substraten wurden diese nach Einwaage in das Schraubdeckelglas zunächst mit dem entsprechenden Lösungsmittel verdünnt und anschließend Cu(OAc)₂ hinzugefügt. Das entsprechende Nitril (3.0 mL) wurde als Lösungsmittel hinzugefügt, das Schraubgläschen wurde fest verschlossen und bei Raumtemperatur 1 min kräftig gerührt, um die Reaktionsbestandteile gut zu suspendieren. Anschließend wurde das Reaktionsgefäß in einen auf 110 bzw. 120 °C vorgeheizten Metallblock gesetzt, dessen Temperatur über ein weiteres mit Öl gefülltes Gewindegläschen unter Rühren kontrolliert und gesteuert wurde, und für 14 - 24 h gerührt. Die Reaktionsmischung wurde aus dem Metallblock entfernt und über einen Zeitraum von ca. 15 min unter Rühren auf Raumtemperatur abgekühlt. Zu diesem Zeitpunkt wurden Umsatz und Erfolg der Reaktion sowie Nebenproduktbildung durch Reaktionskontrolle per ESI-MS, HPLC-MS, GC-MS und/oder DC (je nach Substrat und Produkt) überprüft. Die Reaktionsmischung wurde mit EtOAc (20 mL) verdünnt und Metallsalz-Niederschläge durch gründliches Rühren gut suspendiert. Anschließend wurde die Reaktionsmischung durch Kieselguhr und Kieselgel filtriert (Frittendurchmesser: ca. 1.5 cm; gefüllt mit ca. 1 cm Seesand, 2 cm Kieselgel, 1 cm Seesand, 1 cm Kieselguhr, 1 cm Seesand; zuvor mit ca. 50 ml EtOAc eluiert), der Rückstand wurde sorgfältig mit EtOAc (4 × 20 mL) gewaschen, und die vereinten Filtrate wurden am Rotationsverdampfer unter vermindertem Druck vom Lösungsmittel befreit. Das Rohprodukt wurde im Hochvakuum getrocknet, wobei auf die Flüchtigkeit des Produktes zu achten ist (insbesondere bei 3,5-dimethyl-substituierten Produkten), und ggf. NMR-spektroskopisch untersucht. Das Rohprodukt wurde in CH₂Cl₂ (ca. 5 mL) gelöst, auf Kieselgel (ca. 2 g) adsorbiert und säulenchromatographisch (Kieselgel, Pentan/EtOAc-Gradient) gereinigt.

### Allgemeine Versuchsvorschrift B (AVV B)

In ein über Nacht im Ofen (80 °C) gelagertes Schraubdeckelgläschen (mit PTFE-Dichtungsscheibe, Volumen: ca. 7 mL) mit Magnetrührstäbchen wurde an der Luft das Enamin-Startmaterial (1.0 mmol; (Z)-3-(4-Fluor-phenylamino)-but-2-ensäuremethylester im Fall von Beispiel 1) sowie Cu(OAc)₂ (1.5 mmol) eingewogen. Bei flüssigen oder öligen Substraten wurden diese nach Einwaage in das Schraubdeckelglas zunächst mit dem entsprechenden Lösungsmittel verdünnt und anschließend Cu(OAc)₂ hinzugefügt. Das entsprechende Nitril (1.5 mL) wurde hinzugefügt, das Schraubgläschen wurde fest verschlossen und bei Raumtemperatur 1 min kräftig gerührt, um die Reaktionsbestandteile gut zu suspendieren. Anschließend wurde das Reaktionsgefäß in einen auf 110 °C vorgeheizten Metallblock gesetzt, dessen Temperatur über ein weiteres mit Öl gefülltes Gewindegläschen unter Rühren kontrolliert und gesteuert wurde, und für 24 h gerührt. Die Reaktionsmischung wurde aus dem Metallblock entfernt und über einen Zeitraum von ca. 15 min unter Rühren auf Raumtemperatur abgekühlt. Zu diesem Zeitpunkt wurden Umsatz und Erfolg der Reaktion sowie Nebenproduktbildung durch Reaktionskontrolle per ESI-MS, HPLC-MS, GC-MS und/oder DC (je nach Substrat und Produkt) überprüft. Die Reaktionsmischung wurde mit EtOAc (20 mL) verdünnt und Metallsalz-Niederschläge durch gründliches Rühren gut suspendiert. Anschließend wurde die Reaktionsmischung durch Kieselguhr und Kieselgel filtriert (Frittendurchmesser: ca. 1.5 cm; gefüllt mit ca. 1 cm Seesand, 2 cm Kieselgel, 1 cm Seesand, 1 cm Kieselguhr, 1 cm Seesand; zuvor mit ca. 50 ml EtOAc eluiert), der Rückstand wurde sorgfältig mit EtOAc (4 × 20 mL) gewaschen, und die vereinten Filtrate wurden am Rotationsverdampfer unter vermindertem Druck vom Lösungsmittel befreit. Das Rohprodukt wurde im Hochvakuum getrocknet, wobei auf die Flüchtigkeit des Produktes zu achten ist (insbesondere bei 3,5-dimethyl-substituierten Produkten), und ggf. NMR-spektroskopisch untersucht. Das Rohprodukt wurde in CH₂Cl₂ (ca. 5 mL) gelöst, auf Kieselgel (ca. 2 g) adsorbiert und säulenchromatographisch (Kieselgel, Pentan/EtOAc-Gradient) gereinigt.

### Allgemeine Versuchsvorschrift C (AVV C)

In ein über Nacht im Ofen (140 °C) gelagertes Schraubdeckelgläschen (mit PTFE-Dichtungsscheibe, Volumen: ca. 4 mL) mit Magnetrührstäbchen wurde an der Luft das Enamin-Startmaterial (1.0 mmol) sowie Cu(OAc)₂ (1.5 mmol) eingewogen, gefolgt von der Zugabe von DMF (1 ml) und Nitril (3.0 mmol). Bei flüssigen Enaminsubstraten wurden diese nach Einwaage in das Schraubdeckelglas zunächst mit dem entsprechenden Lösungsmittel verdünnt und anschließend Cu(OAc)₂ hinzugefügt. Das Schraubgläschen wurde fest verschlossen und bei Raumtemperatur 1 min kräftig gerührt, um die Reaktionsbestandteile gut zu suspendieren. Anschließend wurde das Reaktionsgefäß in einen auf 110 °C vorgeheizten Metallblock gesetzt und für 24 h gerührt. Die Reaktionsmischung wurde aus dem Metallblock entfernt und über einen Zeitraum von ca. 15 min unter Rühren auf Raumtemperatur abgekühlt. Zu diesem Zeitpunkt wurden Umsatz und Erfolg der Reaktion sowie Nebenproduktbildung durch Reaktionskontrolle per ESI MS, HPLC MS, GC MS und/oder DC (je nach Substrat und Produkt) überprüft. Die Reaktionsmischung wurde mit EtOAc (20 mL) verdünnt und Metallsalz-Niederschläge durch gründliches Rühren gut suspendiert. Anschließend wurde die Reaktionsmischung durch Kieselguhr und Kieselgel filtriert (Frittendurchmesser: ca. 1.5 cm; gefüllt mit ca. 1 cm Seesand, 2 cm Kieselgel, 1 cm Seesand, 2 cm Kieselguhr, 1 cm Seesand; zuvor mit ca. 50 ml EtOAc eluiert), der Rückstand wurde sorgfältig mit EtOAc (4 × 20 mL) gewaschen, und die vereinten Filtrate wurden am Rotationsverdampfer unter vermindertem Druck vom Lösungsmittel befreit. Das Rohprodukt wurde in CH₂Cl₂ (ca. 5 mL) gelöst, auf Kieselgel (ca. 2 g) adsorbiert und säulenchromatographisch (Kieselgel, Pentan/EtOAc-Gradient) gereinigt.

### Allgemeine Versuchsvorschrift D (AVV D)

In ein über Nacht im Ofen (140 °C) gelagertes Schlenkgefäß (Volumen: ca. 10 mL) mit Magnetrührstäbchen wurde an der Luft das Enamin-Startmaterial (0.5 mmol) sowie Cu(OAc)₂ (0.05 mmol) eingewogen, gefolgt von der Zugabe von 1,2-Dichlorethan (DCE, 1 ml), des Nitrils (0.75 mmol) und dann Pivalinsäure (0.025 mmol, zugegeben als 0.4 M Stammlösung in DCE). Anschließend wurde der untere, die Verbindungen enthaltene Teil mit flüssigem Stickstoff eingekühlt, das Gefäß anschließend kurz evakuiert und dann mit Sauerstoff (O₂) gefüllt. Evakuieren und Füllen mit O₂ wurde noch zweimal wiederholt. Das Schlenkgefäß wurde verschlossen und in einem auf 110 °C vorgeheizten Ölbad für 24 h gerührt. Die Reaktionsmischung wurde aus dem Metallblock entfernt und über einen Zeitraum von ca. 15 min unter Rühren auf Raumtemperatur abgekühlt. Zu diesem Zeitpunkt wurden Umsatz und Erfolg der Reaktion sowie Nebenproduktbildung durch Reaktionskontrolle per ESI MS, HPLC MS, GC MS und/oder DC (je nach Substrat und Produkt) überprüft. Die Reaktionsmischung wurde mit EtOAc (10 mL) verdünnt und Metallsalz-Niederschläge durch gründliches Rühren gut suspendiert. Anschließend wurde die Reaktionsmischung durch Kieselguhr und Kieselgel filtriert (Frittendurchmesser: ca. 1.5 cm; gefüllt mit ca. 0.5 cm Seesand, 1 cm Kieselguhr, 0.5 cm Seesand; zuvor mit ca. 10 ml EtOAc eluiert), der Rückstand wurde sorgfältig mit EtOAc (4 × 10 mL) gewaschen, und die vereinten Filtrate wurden am Rotationsverdampfer unter vermindertem Druck vom Lösungsmittel befreit. Das Rohprodukt wurde in CH₂Cl₂ (ca. 5 mL) gelöst, auf Kieselgel (ca. 2 g) adsorbiert und säulenchromatographisch (Kieselgel, Pentan/EtOAc-Gradient) gereinigt.

### Beispiele:

### Beispiel 1: Darstellung von 1-(4-Fluorphenyl)-3,5-dimethyl-1H-pyrazol-4-carbonsäuremethylester

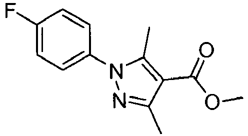

Gemäß AVV A wurde (Z)-3-(4-Fluorphenylamino)-but-2-ensäuremethylester (209.2 mg, 1.0 mmol, 1.0 Äquiv.) mit Cu(OAc)₂ (544.9 mg, 3.0 mmol, 3.0 Äquiv.) in Acetonitril (3.0 mL, 57.1 mmol, 57.1 Äquiv.) 24 h bei 110 °C gerührt. Nach säulenchromatographischer Reinigung (Kieselgel (50 g), Pentan/EtOAc 95:5 → 0:100) wurde das Produkt als weißer Feststoff erhalten (216.6 mg, 0.87 mmol, 87%).
Alternativ wurde 1-(4-Fluorphenyl)-3,5-dimethyl-1*H*-pyrazol-4-carbonsäuremethylester gemäß AVV B in einer Ausbeute von 84% erhalten.

### Beispiel 2: Darstellung von 1-(4-Fluorphenyl)-3-trideuteromethyl-5-methyl-1H-pyrazol-4-carbonsäuremethylester

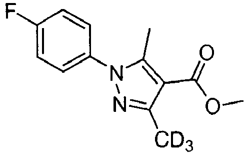

Gemäß AVV A wurde (Z)-3-(4-Fluorphenylamino)-but-2-ensäuremethylester (209.2 mg, 1.0 mmol, 1.0 Äquiv.) mit Cu(OAc)₂ (544.9 mg, 3.0 mmol, 3.0 Äquiv.) in *d³*-Acetonitril (3.0 mL, 57.5 mmol, 57.5 Äquiv.) 24 h bei 110 °C gerührt. Nach säulenchromatographischer Reinigung (Kieselgel (50 g), Pentan/EtOAc 95:5 → 0:100) wurde das Produkt als weißer Feststoff erhalten (212.2 mg, 0.84 mmol, 84%).
Alternativ wurde 1-(4-Fluorphenyl)-3-trideuteromethyl-5-methyl-1*H*-pyrazol-4-carbonsäuremethylester gemäß AVV B in einer Ausbeute von 85% erhalten.

### Beispiel 3: Darstellung von 3-Ethyl-1-(4-fluorphenyl)-5-methyl-1H-pyrazol-4-carbonsäuremethylester

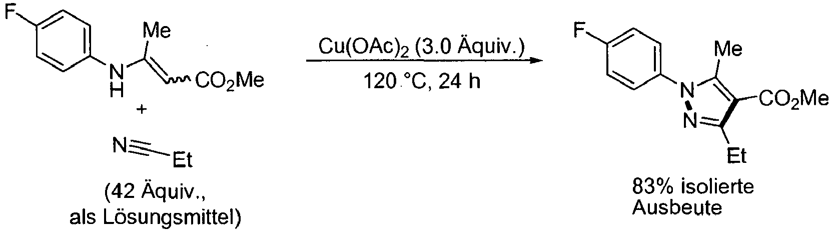

Gemäß AVV A wurde (Z)-3-(4-Fluorphenylamino)-but-2-ensäuremethylester (209.2 mg, 1.0 mmol, 1.0 Äquiv.) mit Cu(OAc)₂ (544.9 mg, 3.0 mmol, 3.0 Äquiv.) in Propionitril (3.0 mL, 41.9 mmol, 41.9 Äquiv.) 24 h bei 120 °C gerührt. Nach säulenchromatographischer Reinigung (Kieselgel (50 g), Pentan/EtOAc 98:2 → 70:30 → 0:100) wurde das Produkt als weißer Feststoff erhalten (218.2 mg, 0.83 mmol, 83%).
Alternativ wurde 3-Ethyl-1-(4-fluorphenyl)-5-methyl-1*H*-pyrazol-4-carbonsäuremethylester gemäß AVV B in einer Ausbeute von 77% erhalten.

### Beispiel 4: Darstellung von 1-(4-Fluorphenyl)-3-isopropyl-5-methyl-1H-pyrazol-4-carbonsäuremethylester

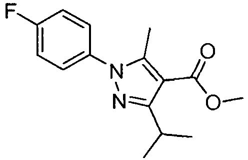

Gemäß AVV A wurde (Z)-3-(4-Fluorphenylamino)-but-2-ensäuremethylester (209.2 mg, 1.0 mmol, 1.0 Äquiv.) mit Cu(OAc)₂ (544.9 mg, 3.0 mmol, 3.0 Äquiv.) in Isobutyronitril (3.0 mL, 33.0 mmol, 33.0 Äquiv.) 24 h bei 110 °C gerührt. Nach säulenchromatographischer Reinigung (Kieselgel (50 g), Pentan/EtOAc 98:2 → 0:100) wurde das Produkt als weißer Feststoff erhalten (195.3 mg, 0.71 mmol, 71%).
Alternativ wurde 1-(4-Fluorphenyl)-3-isopropyl-5-methyl-1*H*-pyrazol-4-carbonsäuremethylester gemäß AVV B in einer Ausbeute von 60% erhalten.

### Beispiel 5: Darstellung von 1-(4-Fluorphenyl)-5-methyl-3-phenyl-1H-pyrazol-4-carbonsäuremethylester

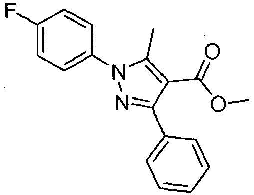

Gemäß AVV A wurde (Z)-3-(4-Fluorphenylamino)-but-2-ensäuremethylester (209.2 mg, 1.0 mmol, 1.0 Äquiv.) mit Cu(OAc)₂ (544.9 mg, 3.0 mmol, 3.0 Äquiv.) in Benzonitril (3.0 mL, 29.4 mmol, 29.4 Äquiv.) 23 h bei 120 °C gerührt. Nach säulenchromatographischer Reinigung (Kieselgel (50 g), Pentan/EtOAc 98:2 → 0:100) wurde das Produkt als weißer Feststoff erhalten (279.0 mg, 0.90 mmol, 90%).
Alternativ wurde 1-(4-Fluorphenyl)-5-methyl-3-phenyl-1*H*-pyrazol-4-carbonsäuremethylester gemäß AVV B in einer Ausbeute von 80% erhalten.

### Beispiel 6: Darstellung von 1-(4-Ethoxyphenyl)-3-ethyl-5-methyl-1H-pyrazol-4-carbonsäuremethylester

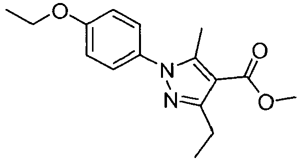

Gemäß AVV A wurde (Z)-3-(4-Ethoxyphenylamino)-but-2-ensäuremethylester (235.3 mg, 1.0 mmol, 1.0 Äquiv.) mit Cu(OAc)₂ (544.9 mg, 3.0 mmol, 3.0 Äquiv.) in Propionitril (3.0 mL, 41.9 mmol, 41.9 Äquiv.) 24 h bei 120 °C gerührt. Das bläulich-braune Rohprodukt (307.8 mg) wurde säulenchromatographisch gereinigt (Kieselgel (50 g), Pentan/EtOAc 98:2→ 70:30 → 0:100), wodurch das reine Produkt als schwach-gelbes Öl erhalten wurde (254.7 mg, 0.88 mmol, 88%).
Alternativ wurde 1-(4-Ethoxyphenyl)-3-ethyl-5-methyl-1*H*-pyrazol-4-carbonsäuremethylester gemäß AVV B in einer Ausbeute von 83% erhalten.

### Beispiel 7: Darstellung von 1-(4-Ethoxyphenyl)-5-methyl-3-phenyl-1H pyrazo 1-4-carbonsäuremethylester

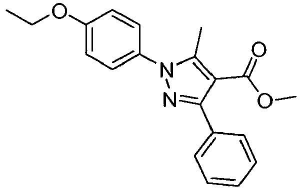

Gemäß AVV A wurde (Z)-3-(4-Ethoxyphenylamino)-but-2-ensäuremethylester (235.3 mg, 1.0 mmol, 1.0 Äquiv.) mit Cu(OAc)₂ (544.9 mg, 3.0 mmol, 3.0 Äquiv.) in Benzonitril (3.0 mL, 29.4 mmol, 29.4 Äquiv.) 14 h bei 120 °C gerührt. Das olivgrüne ölige Rohprodukt (490.3 mg) wurde säulenchromatographisch gereinigt (Kieselgel (50 g), Pentan/EtOAc 95:5 → 0:100). Das gewünschte Produkt wurde als weißer Feststoff erhalten (291.0 mg, 0.87 mmol, 87%).

### Beispiel 8: Darstellung von 3-Ethyl-5-methyl-1-(4-nitrophenyl)-1H-pyrazol-4-carbonsäuremethylester

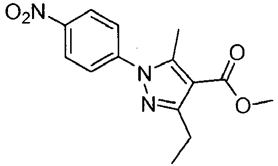

Gemäß AVV A wurde (Z)-3-(4-Nitro-phenylamino)-but-2-ensäuremethylester (236.2 mg, 1.0 mmol, 1.0 Äquiv.) mit Cu(OAc)₂ (544.9 mg, 3.0 mmol, 3.0 Äquiv.) in Propionitril (3.0 mL, 41.9 mmol, 41.9 Äquiv.) 21 h bei 120 °C gerührt. Der als Rohprodukt erhaltene grüne Feststoff (293.5 mg) wurde säulenchromatographisch gereinigt (Kieselgel (50 g), CH₂Cl₂/EtOAc 100:0→ 90:10 → 0:100), wodurch das reine Produkt als leicht gelblicher Feststoff erhalten wurde (188.3 mg, 0.65 mmol, 65%).

### Beispiel 9: Darstellung von 5-Methyl-1-(4-nitrophenyl)-3-phenyl-1H-pyrazol-4-carbonsäuremethylester

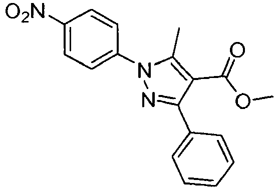

Gemäß AVV A wurde (Z)-3-(4-Nitro-phenylamino)-but-2-ensäuremethylester (236.2 mg, 1.0 mmol, 1.0 Äquiv.) mit Cu(OAc)₂ (544.9 mg, 3.0 mmol, 3.0 Äquiv.) in Benzonitril (3.0 mL, 29.4 mmol, 29.4 Äquiv.) 14 h bei 120 °C gerührt. Der als Rohprodukt erhaltene braune Feststoff (533.2 mg) wurde säulenchromatographisch gereinigt (Kieselgel (50 g), CH₂Cl₂/EtOAc 100:0→ 90:10 → 0:100), wodurch das reine Produkt als leicht gelblicher Feststoff erhalten wurde (253.5 mg, 0.75 mmol, 75%).

### Beispiel 10: Darstellung von 3-Ethyl-5-methyl-1-(2,4,6-trimethylphenyl)-1H-pyrazol-carbonsäureethylester

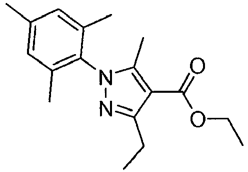

Gemäß AVV A wurde (Z)-3-(2,4,6-Trimethylphenyl-amino)-but-2-ensäureethylester (247.3 mg, 1.0 mmol, 1.0 Äquiv.) mit Cu(OAc)₂ (544.9 mg, 3.0 mmol, 3.0 Äquiv.) in Propionitril (3.0 mL, 41.9 mmol, 41.9 Äquiv.) 20 h bei 120 °C gerührt. Das als Rohprodukt erhaltende dunkelgrüne Öl (333.3 mg) wurde säulenchromatographisch gereinigt (Kieselgel (25 g), Pentan/EtOAc 98:2→ 0:100). Das Produkt wurde als gelbliches Öl erhalten (275.3 mg, 0.92 mmol, 92%).

### Beispiel 11: Darstellung von 5-Methyl-3-phenyl-1-(2,4,6-trimethylphenyl)-1H-pyrazol-4-carbonsäureethylester

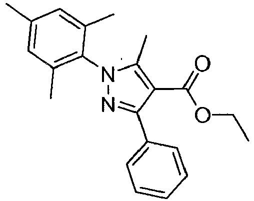

Gemäß AVV A wurde (Z)-3-(2,4,6-Trimethylphenyl-amino)-but-2-ensäureethylester (247.3 mg, 1.0 mmol, 1.0 Äquiv.) mit Cu(OAc)₂ (544.9 mg, 3.0 mmol, 3.0 Äquiv.) in Benzonitril (3.0 mL, 29.4 mmol, 29.4 Äquiv.) 14 h bei 120 °C gerührt. Das als Rohprodukt erhaltende braune Öl (524.3 mg) wurde säulenchromatographisch gereinigt (Kieselgel (50 g), Pentan/EtOAc 98:2→ 0:100). Das Produkt wurde als weißer Feststoff erhalten (289.4 mg, 0.83 mmol, 83%).

### Beispiel 12: Darstellung von 3-Methyl-1-phenyl-1H-pyrazol-4-carbonsäuremethylester

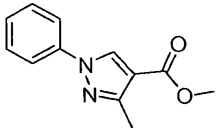

Gemäß AVV A wurde 3-Phenylamino-acrylsäuremethylester (**197**) (177.2 mg, 1.0 mmol, 1.0 Äquiv.) mit Cu(OAc)₂ (544.9 mg, 3.0 mmol, 3.0 Äquiv.) in Acetonitril (3.0 mL, 57.1 mmol, 57.1 Äquiv.) 22 h bei 110 °C gerührt. Der als Rohprodukt erhaltene grünliche Feststoff (242.4 mg) wurde säulenchromatographisch gereinigt (Kieselgel (50 g), Pentan/MTBE 95:5→ 0:100, dann MTBE/EtOAc 100:0→ 0:100), wodurch das gewünschte Produkt als gelblicher Feststoff erhalten wurde (35.1 mg, 0.16 mmol, 16%).

### Beispiel 13: Darstellung von 3-Ethyl-1-phenyl-1H-pyrazol-4-carbonsäuremethylester

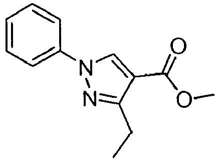

Gemäß AVV A wurde 3-Phenylamino-acrylsäuremethylester (177.2 mg, 1.0 mmol, 1.0 Äquiv.) mit Cu(OAc)₂ (544.9 mg, 3.0 mmol, 3.0 Äquiv.) in Propionitril (3.0 mL, 41.9 mmol, 41.9 Äquiv.) 19 h bei 120 °C gerührt. Das orange-braune ölige Rohprodukt (191.2 mg) wurde säulenchromatographisch gereinigt (Kieselgel (50 g), Pentan/EtOAc 98:2→ 0:100), wodurch das reine Produkt als weißer Feststoff erhalten wurde (37.2 mg, 0.16 mmol, 16%).

### Beispiel 14: Darstellung von 1,3-Diphenyl-1H-pyrazol-4-carbonsäuremethylester

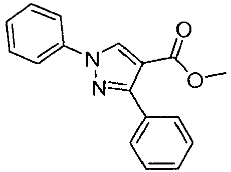

Gemäß AVV A wurde 3-Phenylamino-acrylsäuremethylester (177.2 mg, 1.0 mmol, 1.0 Äquiv.) mit Cu(OAc)₂ (544.9 mg, 3.0 mmol, 3.0 Äquiv.) in Benzonitril (3.0 mL, 29.4 mmol, 41.9 Äquiv.) 14 h bei 120 °C gerührt. Der als Rohprodukt erhaltene grüne Feststoff (438.1 mg) wurde säulenchromatographisch gereinigt (Kieselgel (50 g), Pentan/MTBE 95:5→ 0:100, dann MTBE/EtOAc 100:0→ 0:100). Das gewünschte Produkt wurde als weißer Feststoff erhalten (108.8 mg, 0.39 mmol, 39%).

### Beispiel 15: Darstellung von-3-Methyl-1,5-diphenyl-1H-pyrazol-4-carbonsäureethylester

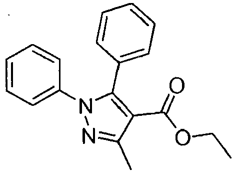

Gemäß AVV A wurde (Z)-3-Phenyl-3-phenylamino-acrylsäureethylester (267.3 mg, 1.0 mmol, 1.0 Äquiv.) mit Cu(OAc)₂ (544.9 mg, 3.0 mmol, 3.0 Äquiv.) in Acetonitril (3.0 mL, 57.1 mmol, 57.1 Äquiv.) 21 h bei 110 °C gerührt. Das blau-grüne Rohprodukt (389.3 mg) wurde säulenchromatographisch gereinigt (Kieselgel (50 g), Pentan/EtOAc 98:2→ 0:100). Das Produkt wurde als weißer Feststoff erhalten (269.3 mg, 0.88 mmol, 88%).

### Beispiel 16: Darstellung von 3-Ethyl-1,5-diphenyl-1H-pyrazol-4-carbonsäureethylester

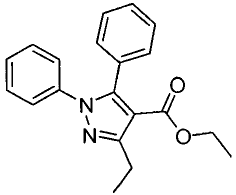

Gemäß AVV A wurde (Z)-3-Phenyl-3-phenylamino-acrylsäureethylester (267.3 mg, 1.0 mmol, 1.0 Äquiv.) mit Cu(OAc)₂ (544.9 mg, 3.0 mmol, 3.0 Äquiv.) in Propionitril (3.0 mL, 41.9 mmol, 41.9 Äquiv.) 24 h bei 120 °C gerührt. Das dunkelbraun-blaue Rohprodukt (342.3 mg) wurde säulenchromatographisch gereinigt (Kieselgel (50 g), Pentan/EtOAc 98:2→ 80:20 → 0:100). Das Produkt wurde als weißer kristalliner Feststoff erhalten (278.1 mg, 0.87 mmol, 87%).
Alternativ wurde 3-Ethyl-1,5-diphenyl-1*H*-pyrazol-4-carbonsäureethylester gemäß AVV B in einer Ausbeute von 80% erhalten.

### Beispiel 17: Darstellung von 1,3,5-Triphenyl-1H-pyrazol-4-carbonsäureethylester

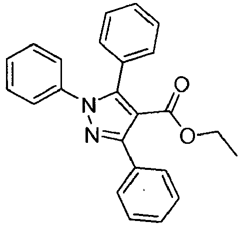

Gemäß AVV A wurde (Z)-3-Phenyl-3-phenylamino-acrylsäureethylester (267.3 mg, 1.0 mmol, 1.0 Äquiv.) mit Cu(OAc)₂ (544.9 mg, 3.0 mmol, 3.0 Äquiv.) in Benzonitril (3.0 mL, 29.4 mmol, 29.4 Äquiv.) 14 h bei 120 °C gerührt. Der als Rohprodukt erhaltene grüne Feststoff (458.2 mg) wurde säulenchromatographisch gereinigt (Kieselgel (50 g), Pentan/EtOAc 95:5 → 0:100). Das Produkt wurde als weißer Feststoff erhalten (302.3 mg, 0.82 mmol, 82%).

### Beispiel 18: Darstellung von 3-Ethyl-1-(4-fluorphenyl)-5-methyl-1H-pyrazol-4-carbonsäure-tert-butylester

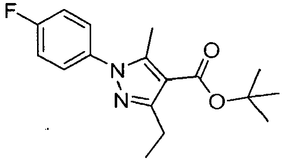

Gemäß AVV A wurde (*Z*)-3-(4-Fluorphenylamino)-but-2-ensäure-tert-butylester (251.3 mg, 1.0 mmol, 1.0 Äquiv.) mit Cu(OAc)₂ (544.9 mg, 3.0 mmol, 3.0 Äquiv.) in Propionitril (3.0 mL, 41.9 mmol, 41.9 Äquiv.) 24 h bei 120 °C gerührt. Das dunkelbraun-blaue Rohprodukt (295.9 mg) wurde mittels ¹H- und ¹⁹F-NMR-Spektroskopie untersucht. ¹⁹F-NMR-Spektren zeigten das gewünschte Produkt (δ = -112.7 ppm) und *N*-(4-Fluorphenyl)-acetamid (δ = -118.0 ppm) im Verhältnis 89:11. Nach säulenchromatographischer Reinigung (Kieselgel (50 g), Pentan/EtOAc 98:2 → 70:30 → 0:100) wurde das Produkt als gelbliches Öl erhalten (222.9 mg, 0.73 mmol, 73%).

### Beispiel 19: Darstellung von 3-Ethyl-1-(4-fluorphenyl)-5-methyl-1H-pyrazol-4-carbonsäurebenzylester

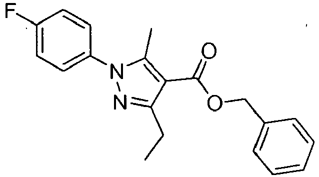

Gemäß AVV A wurde (Z)-3-(4-Fluorphenylamino)-but-2-ensäurebenzylester (285.3 mg, 1.0 mmol, 1.0 Äquiv.) mit Cu(OAc)₂ (544.9 mg, 3.0 mmol, 3.0 Äquiv.) in Propionitril (3.0 mL, 41.9 mmol, 41.9 Äquiv.) 24 h bei 120 °C gerührt. Nach säulenchromatographischer Reinigung (Kieselgel (50 g), Pentan/EtOAc 98:2→ 70:30 → 0:100) wurde das Produkt als gelbes Öl erhalten (283.0 mg, 0.84 mmol, 84%).

### Beispiel 20: Darstellung von (3-Ethyl-5-methyl-1-phenyl-1H-pyrazol-4-yl)-phenylmethanon

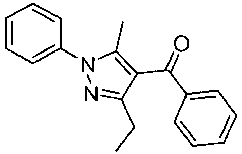

Gemäß AVV A wurde (*Z*)-1-Phenyl-3-phenylamino-but-2-en-1-on (237.3 mg, 1.0 mmol, 1.0 Äquiv.) mit Cu(OAc)₂ (544.9 mg, 3.0 mmol, 3.0 Äquiv.) in Propionitril (3.0 mL, 41.9 mmol, 41.9 Äquiv.) 24 h bei 120 °C gerührt. Nach säulenchromatographischer Reinigung (Kieselgel (50 g), Pentan/EtOAc 98:2→ 60:40 → 0:100) des dunkelbraunbläulichen Rohprodukts (330.0 mg) wurde das gewünschte Produkt als gelbes Öl erhalten (126.2 mg, 0.43 mmol, 43%).

### Beispiel 21: Darstellung von 1-(3-Ethyl-5-methyl-1-phenyl-1H-pyrazol-4-yl)-ethanon

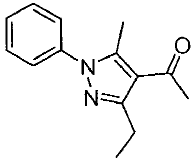

Gemäß AVV A wurde (Z)-4-Phenylaminopent-3-en-2-on (175.2 mg, 1.0 mmol, 1.0 Äquiv.) mit Cu(OAc)₂ (544.9 mg, 3.0 mmol, 3.0 Äquiv.) in Propionitril (3.0 mL, 41.9 mmol, 41.9 Äquiv.) 24 h bei 120 °C gerührt. Nach säulenchromatographischer Reinigung (Kieselgel (50 g), Pentan/EtOAc 94:6→ 0:100) des braunen öligen Rohprodukts (157.3 mg) wurde das gewünschte Produkt als gelbes Öl erhalten (57.2 mg, 0.25 mmol, 25%).

### Beispiel 22: Darstellung von 3-Ethyl-5-methyl-1-phenethyl-1H-pyrazol-4-carbonsäuremethylester

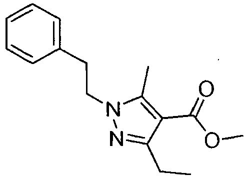

Gemäß AVV A wurde (Z)-3-Phenethylamino-but-2-ensäuremethylester (219.3 mg, 1.0 mmol, 1.0 Äquiv.) mit Cu(OAc)₂ (544.9 mg, 3.0 mmol, 3.0 Äquiv.) in Propionitril (3.0 mL, 41.9 mmol, 41.9 Äquiv.) 24 h bei 120 °C gerührt. Das nach der Aufarbeitung als Rohprodukt erhaltene dunkelbraune Öl (295.5 mg) wurde säulenchromatographisch gereinigt (Kieselgel (50 g), Pentan/EtOAc 98:2→ 70:30 → 0:100). Das gewünschte Produkt wurde als gelbes Öl erhalten (157.8 mg, 0.58 mmol, 58%).

### Beispiel 23: Darstellung von 1-(1-(4-(Methoxycarbonyl)-3-ethyl-5-methyl-1H-pyrazol-1-yl)naphthalin-5-yl)-3-ethyl-5-methyl-1H-pyrazol-4-carbonsäuremethylester

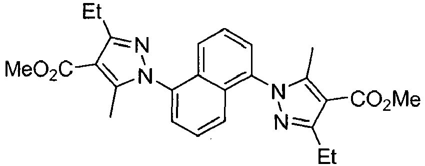

Gemäß AVV A wurde (*Z*)-3-[5-((*Z*)-2-Methoxycarbonyl-1-methyl-vinylamino)-naphthalen-1-ylamino]-but-2-ensäuremethylester (354.4 mg, 1.0 mmol, 1.0 Äquiv.) mit Cu(OAc)₂ (1.09 g, 6.0 mmol, 6.0 Äquiv.) in Propionitril (6.0 mL, 83.9 mmol, 83.9 Äquiv.) 16 h bei 120 °C gerührt. Der nach der Aufarbeitung als Rohprodukt erhaltene grün-weiße Feststoff (474.5 mg) wurde säulenchromatographisch gereinigt (Kieselgel (50 g), Pentan/EtOAc 93:7→ 0:100). Das gewünschte Produkt wurde als weißer Feststoff erhalten (270.1 mg, 0.59 mmol, 59%). Desweiteren wurde eine Fraktion mit leicht verunreinigtem Produkt als gelblicher Feststoff isoliert (58.4 mg, 0.14 mmol, 14%).

Beispiel 24: Darstellung von 1-(3-(4-(Methoxycarbonyl)-3-ethyl-5-methyl-1*H*-pyrazol-1-yl)phenyl)-3-ethyl-5-methyl-1*H*-pyrazol-4-carbonsäuremethylester

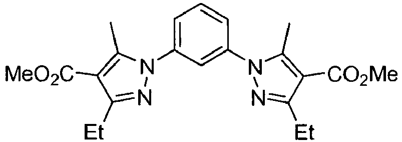

Gemäß AVV A wurde (*Z*)-3-[3-((*Z*)-2-Methoxy-carbonyl-1-methyl-vinylamino)-phenylamino]-buten-äuremethylester (**213**) (304.3 mg, 1.0 mmol, 1.0 Äquiv.) mit Cu(OAc)₂ (1.09 g, 6.0 mmol, 6.0 Äquiv.) in Propionitril (6.0 mL, 83.9 mmol, 83.9 Äquiv.) 24 h bei 110 °C gerührt. Der nach der Aufarbeitung als Rohprodukt erhaltene braune Feststoff (383.8 mg) wurde säulenchromatographisch gereinigt (Kieselgel (50 g), Pentan/EtOAc 94:6→ 0:100). Das gewünschte Produkt wurde als weißer Feststoff erhalten (224.7 mg, 0.55 mmol, 55%). Desweiteren wurde eine Fraktion mit leicht verunreinigtem Produkt als gelblicher Feststoff isoliert (63.1 mg, 0.15 mmol, 15%).

### Beispiel 25: Darstellung von 3,5-Dimethyl-1-phenyl-1H-pyrazol-4-carbonsäureethylester durch Ein-Topf-Pyrazolsynthese aus Anilin und Methylacetoacetat im 5.0 mmol-Maßstab mit InBr₃ als katalytischer Lewissäure

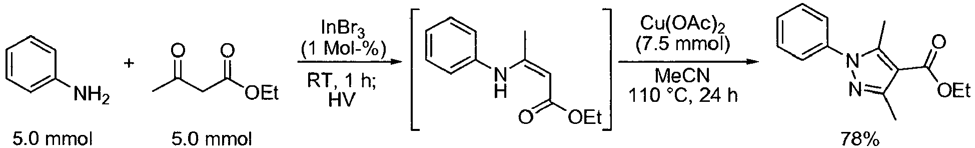

In einen zuvor im Hochvakuuum ausgeheizten Langhalskolben mit Magnetrührstäbchen wurde an der Luft InBr₃ (17.7 mg, 0.05 mmol, 1 Mol-%) eingewogen, das Gefäß wurde evakuiert und mit Argon geflutet. Im Argongegenstrom wurde trockenes Anilin (456 µL, 5.0 mmol, 1.0 Äquiv.) und Acetessigsäureethylester (632 µL, 5.0 mmol, 1.0 Äquiv.) zugegeben und die Reaktionsmischung wurde 1 h bei Raumtemperatur gerührt. Um gebildetes Reaktionswasser zu entfernen wurde die Reaktionsmischung evakuiert (Hochvakuum) und das Reaktionsgefäß durch Eintauchen in ein Bad aus flüssigem Stickstoff (- 196 °C) abgekühlt. Nach ungefähr 10 Minuten wurde das Kühlbad entfernt und die Probe konnte sich auf Raumtemperatur erwärmen. Dieser Prozess (Abkühlen und Auftauen) wurde drei Mal wiederholt. Anschließend wurde die Probe noch für 12 h unter Hochvakuum bei Raumtemperatur gehalten und dann mit Argon geflutet.

Trockenes Acetonitril (7.5 mL, 142.7 mmol, 28.6 Äquiv.) sowie Cu(OAc)₂ (1.362 g, 7.5 mmol, 1.5 Äquiv.) wurden unter Argon hinzugefügt, die Mischung wurde 1 min bei Raumtemperatur kräftig gerührt, das Druckgefäß wurde verschlossen und die Reaktionsmischung wurde in einem vorgeheizten Ölbad 24 h bei 110 °C gerührt. Die Mischung wurde auf Raumtemperatur abgekühlt, mit 50 mL EtOAc verdünnt und durch Kieselguhr und Kieselgel filtriert. Der Rückstand wurde sorgfältig mit EtOAc (3 × 20 mL) gewaschen. Die vereinten Filtrate wurden am Rotationsverdampfer unter vermindertem Druck vom Lösungsmittel befreit und das Rohprodukt wurde 10 min im Hochvakuum getrocknet. Das erhaltene Rohprodukt wurde in CH₂Cl₂ (20 mL) gelöst, auf Kieselgel (5 g) adsorbiert und säulenchromatographisch (Kieselgel (50 g), Pentan/EtOAc 95:5→ 0:100) gereinigt. Das gewünschte Produkt (3,5-Dimethyl-1-phenyl-1*H*-pyrazol-4-carbonsäureethylester) wurde als gelblicher Feststoff erhalten (948.9 mg, 3.88 mmol, 78%) und stimmte in seinen analytisch erhaltenen Daten mit zuvor erhaltenen und den Literaturdaten überein.

### Beispiel 26: Darstellung von 5-Methyl-1,3-diphenyl-1H-pyrazol-4-carbonsäureethylester durch Ein-Topf-Pyrazolsynthese aus Anilin und Methylacetoacetat im 5.0 mmol-Maßstab mit InBr₃ als katalytischer Lewissäure

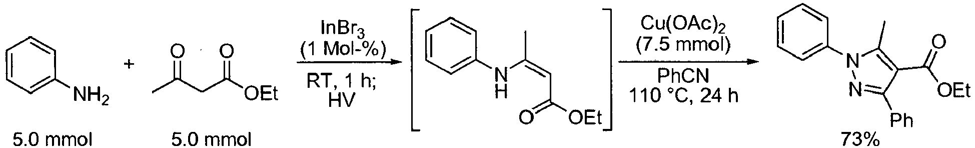

Nach der Vorschrift von Beispiel 25, allerdings unter Verwendung von Benzonitril (7.5 mL) statt Acetonitril, wurde das gewünschte Produkt (5-Methyl-1,3-diphenyl-1*H*-pyrazol-4-carbonsäureethylester) in 73% (948.3 mg, 3.67 mmol) Ausbeute erhalten.

### Beispiele 27 bis 37:

Beispielverbindungen 27 bis 37 wurden gemäß AAV C hergestellt. Dabei handelt es sich um folgende Verbindungen:
Beispiel 27: 5-Methyl-1,3-diphenyl-1H-pyrazol-4-carbonsäuremethylester [Anmerkung: Als Enamin-Startmaterial wurde (Z)-3-(Phenylamino)-but-2-ensäuremethylester verwendet]
Beispiel 28: 5-Methyl-1-phenyl-3-(m-tolyl)-1H-pyrazol-4-carbonsäuremethylester
Beispiel 29: 3-(4-Acetylphenyl)-5-methyl-1-phenyl-1H-pyrazol-4-carbonsäuremethylester
Beispiel 30: 5-Methyl-3-(3-methylpyridin-2-yl)-1-phenyl-1H-pyrazol-4-carbonsäuremethylester
Beispiel 31: 3-(Furan-2-yl)-5-methyl-1-phenyl-1H-pyrazol-4-carbonsäuremethylester
Beispiel 32: 5-Methyl-1-phenyl-3-styryl-1H-pyrazol-4-carbonsäuremethylester
Beispiel 33 : 1,5-Dimethyl-3-phenyl-1H-pyrazol-4-carbonsäuremethylester
Beispiel 34: 1-Butyl-5-methyl-3-phenyl-1H-pyrazol-4-carbonsäuremethylester [Anmerkung: Das Enaminausgangsmaterial ist flüssig]
Beispiel 35: 1-Isopropyl-5-methyl-3-phenyl-1H-pyrazol-4-carbonsäuremethylester [Anmerkung: Das Enaminausgangsmaterial ist flüssig]
Beispiel 36: 3-Ethyl-5-methyl-1-phenyl-1H-pyrazol-4-carbonsäuremethylester
Beispiel 37: 5-Methyl-1-phenyl-3-(trifluoromethyl)-1H-pyrazol-4-carbonsäuremethylester

Die Ausbeuten und Strukturformeln der Produkte sind in der folgenden Tabelle 1 angegeben

**Tabelle I:**

| Beispiel | Pyraz | Ausbeute (%)^{a} | Beispiel | Pyrazole | Ausbeute (%)^{a} |
|---|---|---|---|---|---|
| 27 | 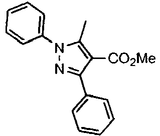 ole | 72 (213.1 mg, gelber Feststoff) | 33 | 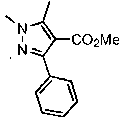 | *72^{b}* (166.0 mg, gelber Feststoff) |
| 28 | 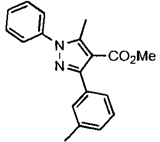 | 61 (185.5 mg, gelber Feststoff) | 34 | 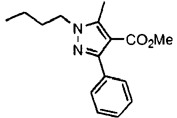 | 48 60^{b} (162.7 mg,^{b} gelbes Öl) |
| 29 | 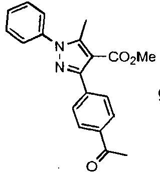 | 84 (281.2 mg, gelblicher Feststoff) | 35 | 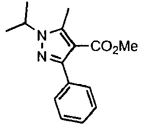 | 25 31^{b} (79.3 mg,^{b} gelbes Öl) |
| 30 | 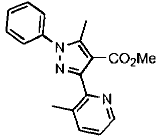 | 65 (198.1 mg, gelber Feststoff) | 36 | 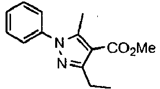 | 52 (126.4 mg, gelber Feststoff) |
| 31 | 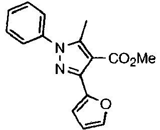 | 77 (218.0 mg, gelber Feststoff) | 37 | 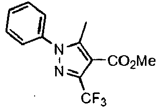 | 45^{c} (32.1 mg, gelber Feststoff) |
| 32 | 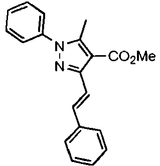 | 19 (60.3 mg, gelbes Öl) | | | |

| | | | | | |
|---|---|---|---|---|---|
| ^{a} Ausbeute an isoliertem Pyrazolprodukt; in Klammern isolierte Menge, Farbe und Konsistenz. *^{b}* Nitril (7 Äquiv.), DMA (*N,N-*Dimethylacetamid) statt DMF als Lösungsmittel. *^{c}* Nach Zugabe aller Substrate und Lösungsmittel in ein Schlenk-Gefäß (Volumen 10 ml für einen 0.25 mmol-Ansatz), wurde der untere, die Verbindungen enthaltene Teil auf 0 °C gekühlt, das Gefäß anschließend evakuiert und dann mit Trifluoracetonitrilgas gefüllt. Beispiele 38 bis 42: | | | | | |

Die Beispiele 38 bis 42 wurden anhand AAV D hergestellt. Dabei handelt es sich um folgende Verbindungen:
Beispiel 38: gleiches Produkt wie Beispiel 27
Beispiel 39: gleiches Produkt wie Beispiel 29
Beispiel 40: gleiches Produkt wie Beispiel 36
Beispiel 41: (5-Methyl-1,3-diphenyl-1H-pyrazol-4-yl)(phenyl)methanon
Beispiel 42: gleiches Produkt wie Beispiel 33

Die Ausbeuten und Strukturformeln der Produkte sind in der folgenden Tabelle II angegeben

**Tabelle II:**

| Beispiel | Pyrazol | Ausbeute (%)^{a} |
|---|---|---|
| 38 | 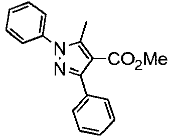 | 53 (77.5 mg, gelblicher Feststoff) |
| 39 | 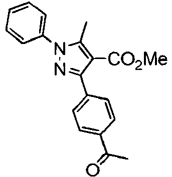 | 69 (115.3 mg, gelblicher Feststoff) |
| 40 | 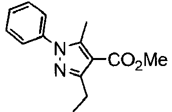 | 29 (35.1 mg, gelber Feststoff) |
| 41 | 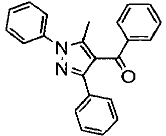 | 48 (81.6 mg, gelblicher Feststoff) |
| 42 | 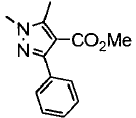 | 42 (47.8 mg, gelber Feststoff) |

| | | |
|---|---|---|
| *^{a}* Ausbeute an isoliertem Pyrazolprodukt; in Klammern isolierte Menge, Farbe und Konsistenz. | | |

Die einzelnen Kombinationen der Bestandteile und der Merkmale von den bereits erwähnten Ausführungen sind exemplarisch; der Austausch und die Substitution dieser Lehren mit anderen Lehren, die in dieser Druckschrift enthalten sind mit den zitierten Druckschriften werden ebenfalls ausdrücklich erwogen. Der Fachmann erkennt, dass Variationen, Modifikationen und andere Ausführungen, die hier beschrieben werden, ebenfalls auftreten können ohne von dem Erfindungsgedanken und dem Umfang der Erfindung abzuweichen. Entsprechend ist die obengenannte Beschreibung beispielhaft und nicht als beschränkend anzusehen. Das in den Ansprüchen verwendetet Wort "umfassen" schließt nicht andere Bestandteile oder Schritte aus. Der unbestimmte Artikel "ein" schließt nicht die Bedeutung eines Plurals aus. Die bloße Tatsache, dass bestimmte Maße in gegenseitig verschiedenen Ansprüchen rezitiert werden, verdeutlicht nicht, dass eine Kombination von diesen Maßen nicht zum Vorteil benutzt werden kann. Der Umfang der Erfindung ist in den folgenden Ansprüchen definiert und den dazugehörigen Äquivalenten.

## Patentansprüche

1. Verfahren zur Herstellung von Pyrazolen durch Umsetzung von Enaminen und N-haltigen Carbonsäurederivaten in Gegenwart eines Oxidationsmittels.

2. Verfahren nach Anspruch 1, wobei die N-haltigen Carbonsäurederivate ausgewählt sind aus der Gruppe enthaltend Nitrile, Carbonsäureamide, Amidine, Imidate, Imidoylchloride, abgeleitete protonierte Salze dieser Verbindungen sowie Mischungen daraus.

3. Verfahren nach Anspruch 1 oder 2, wobei die Umsetzung in Gegenwart einer Lewis-Säure durchgeführt wird.

4. Verfahren nach einem der Ansprüche 1 bis 3, wobei die Umsetzung in Gegenwart von Kupferionen durchgeführt wird

5. Verfahren nach einem der Ansprüche 1 bis 4, wobei als N-haltiges Carbonsäurederivat ein Nitril gewählt wird und die Umsetzung in diesem Nitril als Lösemittel durchgeführt wird

6. Verfahren nach einem der Ansprüche 1 bis 5, wobei das Verhältnis von Enamin zu N-haltigem Carbonsäurederivat von ≥0.2:1 bis ≤150:1 (mol N-haltiges Carbonsäurederivat :mol Enamin) beträgt

7. Verfahren nach einem der Ansprüche 1 bis 6, wobei die Umsetzung in Gegenwart von Cu(II)-Salzen durchgeführt wird und das Verhältnis von Enamin zu Cu(II)-Salz von ≥1.5:1 bis ≤30:1 (mol Cu(II)-Salz :mol Enamin) beträgt.

## Claims

1. Process for the production of pyrazoles by reaction of enamines and N-containing carboxylic acid derivatives in presence of an oxidising agent.

2. Process according to claim 1, wherein the N-containing carboxylic acid derivatives are selected from the group comprising nitriles, carboxylic acid amines, amidines, imidates, imidoyl chlorides, protonated salts derived from these compounds and mixtures thereof.

3. Process according to claim 1 or 2, wherein the reaction is performed in the presence of a Lewis-acid.

4. Process according to any of the claims 1 to 3, wherein the reaction is performed in the presence of copper-ions.

5. Process according to any of the claims 1 to 4, wherein the N-containing carboxylic acid derivative is a nitrile and the conversion is performed in this nitrile as solvent.

6. Process according to any of the claims 1 to 5, wherein the ratio of enamine to N-containing carboxylic acid derivative is from ≥ 0.2:1 to ≤ 150:1 (mol N-containing carboxylic acid derivative: mol enamin).

7. Process according to any of the claims 1 to 6, wherein the reaction is performed in the presence of Cu(II)-salts and the ratio of enamine to Cu(II)-salt is from ≥ 1.5:1 to ≤ 30:1 (mol Cu(II)-salt : mol enamin).

## Revendications

1. Procédé de préparation de pyrazoles par conversion d'énamines et de dérivés d'acides carboxyliques azotés en présence d'un agent oxydant.

2. Procédé selon la revendication 1, dans lequel les dérivés d'acides carboxyliques azotés sont choisis dans le groupe comprenant : les nitriles, les carboxamides, les amidines, les imidates, les chlorures d'imidoyle, les sels protonés dérivés de ces composés ainsi que les mélanges de ceux-ci.

3. Procédé selon la revendication 1 ou la revendication 2, dans lequel la conversion est effectuée en présence d'un acide de Lewis.

4. Procédé selon l'une quelconque des revendications 1 à 3, dans lequel la conversion est effectuée en présence d'ions de cuivre

5. Procédé selon l'une quelconque des revendications 1 à 4, dans lequel un nitrile est choisi comme dérivé d'acide carboxylique azoté et la conversion est effectuée dans ce nitrile comme solvant

6. Procédé selon l'une quelconque des revendications 1 à 5, dans lequel le rapport entre l'énamine et le dérivé d'acide carboxylique azoté est de ≥0,2:1 à ≤150:1 (moles de dérivés d'acides carboxyliques azotés:moles d'énamines)

7. Procédé selon l'une quelconque des revendications 1 à 6, dans lequel la conversion est effectuée en présence de sels de Cu (II), et le rapport entre l'enamine et le sel de cuivre(II) est de ≥1,5:1 à ≤30:1 (moles de sel de Cu (II):moles d'énamine).
